(19) Europäisches Patentamt
European Patent Office
Office européen des brevets

(11) EP 0 625 500 B1

(12) # EUROPÄISCHE PATENTSCHRIFT

(45) Veröffentlichungstag und Bekanntmachung des Hinweises auf die Patenterteilung: **03.09.1997 Patentblatt 1997/36**

(51) Int. Cl.$^6$: **C07C 51/15**, C07C 65/11

(21) Anmeldenummer: **94107478.3**

(22) Anmeldetag: **13.05.1994**

(54) **Verfahren zur Herstellung von aromatischen Hydroxycarbonsäuren**

Process for the preparation of aromatic hydroxycarboxylic acids

Procédé de préparation d'acides aromatiques hydroxycarboxyliques

(84) Benannte Vertragsstaaten:
**CH DE FR GB IT LI**

(30) Priorität: **21.05.1993 DE 4316933**

(43) Veröffentlichungstag der Anmeldung:
**23.11.1994 Patentblatt 1994/47**

(73) Patentinhaber:
**HOECHST AKTIENGESELLSCHAFT**
**65929 Frankfurt am Main (DE)**

(72) Erfinder:
- **Rittner, Siegbert, Dr.**
  **D-64546 Mörfelden (DE)**
- **Rüffer, Hans-Martin, Dr.**
  **D-65719 Hofheim (DE)**
- **Schmid, Jörg, Dr.**
  **D-65817 Eppstein (DE)**
- **Wisser, Thomas, Dr.**
  **D-65552 Limburg (DE)**

(56) Entgegenhaltungen:
**EP-A- 0 053 824**

- **PATENT ABSTRACTS OF JAPAN vol. 6, no. 223 (C-133) (1101) 9. November 1982 & JP-A-57 126 443 (NIPPON SHOKUBAI KAGAKU KOGYO K.K.) 6. August 1982**
- **PATENT ABSTRACTS OF JAPAN vol. 11, no. 273 (C-445) (2720) 4. September 1987 & JP-A-62 077 350 (NIPPON STEEL CORP.) 9. April 1987**
- **PATENT ABSTRACTS OF JAPAN vol. 6, no. 223 (C-133) (1101) 9. November 1982 & JP-A-57 126 443**
- **PATENT ABSTRACTS OF JAPAN vol. 11, no. 273 (C-445) (2720) 4. September 1987 & JP-A-62 077 350**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 625 500 B1

Printed by Rank Xerox (UK) Business Services
2.14.12/3.4

## Beschreibung

Die Erfindung betrifft ein Verfahren zur Herstellung von aromatischen Hydroxycarbonsäuren durch Reaktion eines Alkaliphenolats oder Alkalinaphtholats mit $CO_2$.

Aromatische Hydroxycarbonsäuren, wie 2-Hydroxy-naphthalin-6-carbonsäure, sind wichtige Zwischenprodukte z. B. bei der Herstellung von Farbstoffen, Polyestern, Arzneimitteln und Textilhilfsmitteln (siehe beispielsweise EP-A 0 292 955 und US 4,393,191).

Technisch erfolgt die Synthese der aromatischen Hydroxycarbonsäuren überwiegend nach einer Verfahrensvariante der sogenannten Kolbe-Schmitt-Reaktion. Dabei wird zunächst in einer Vorstufe das Alkalisalz eines Phenolats oder Naphtholats hergestellt durch Reaktion des Phenols oder Naphthols in wäßriger oder organischer Phase mit einer Base, wie KOH oder $K_2CO_3$. Anschließend wird möglichst weitgehend entwässert und das trockene Phenolat oder Naphtholat mit $CO_2$ unter Druck bei 200 bis 300°C umgesetzt (siehe z. B. US 1,593,816, US 4,329,494 und US 4,287,357). Die in diesen Veröffentlichungen beschriebenen Verfahrensvarianten besitzen jedoch einige Nachteile; so entstehen beträchtliche Anteile an Zersetzungsprodukten, wie Teere und Harze, die sich, ebenso wie die oftmals in Nebenreaktionen entstehenden Isomeren, nur schwierig abtrennen lassen. Zusätzlich hat die Synthese nach der Kolbe-Schmitt-Reaktion den Nachteil, daß pro Mol entstandener Hydroxycarbonsäure jeweils ein Mol Phenol bzw. Naphthol in der Reaktionsmasse unumgesetzt zurückbleibt, weswegen die Ausbeute gemäß der Gleichung

2 Mol Alkaliphenolat bzw. -naphtholat + 1 Mol $CO_2$ $\rightarrow$ 1 Mol Bisalkalisalz der Hydroxycarbonsäure
+ 1 Mol Phenol bzw. Naphthol

auf prinzipiell 50 % limitiert ist. Aufgrund der Neigung des freigesetzten Phenols oder Naphthols mit den bereits entstandenen Reaktionsprodukten unerwünschte Folgereaktionen (Bildung von Teeren und Harzen) einzugehen, ergeben sich bei dieser Art der Reaktion zwangsläufig die obengenannten Nachteile.

Zwar wurde für den Fall der Synthese von 2-Hydroxy-naphthalin-6-carbonsäure mit den in EP-A 0 053 824, EP-A 0 081 753 und EP-A 0 254 596 beschriebenen Verfahrensweisen versucht, die Reaktionsführung zu verbessern und die Bildung der Zersetzungsprodukte zu vermindern, indem man in inerten Lösemitteln arbeitet und Naphthol kontinuierlich durch Strippen mit $CO_2$ aus dem Reaktionssystem entfernt. Es bleibt dennoch der Nachteil eines auf 50 % beschränkten theoretischen Umsatzes, der allerdings in der Praxis durch den Zusatz eines alkalischen Metallsalzes, z. B. Alkali- oder Erdalkalicarbonate oder -hydrogencarbonate, zum Reaktionsansatz geringfügig überschritten werden kann.

Ein weiteres Problem stellt die unzureichende thermische Stabilität der Alkaliphenolate und Naphtholate dar, die bei den genannten Reaktionstemperaturen bereits einer nicht unerheblichen Zersetzung unterliegen. Für die nötigen Reaktionszeiten zwischen 3 und 10 Stunden bedeutet das, daß die bisher in der Praxis üblichen Batch-Verfahrensweisen apparativ nicht die beste Lösung darstellen, wenn man sowohl die hohe Temperaturempfindlichkeit als auch die Reaktionsfähigkeit des Phenolats oder Naphtholats im Hinblick auf mögliche Folgereaktionen berücksichtigt. Die während des Reaktionsablaufs noch in hoher Konzentration vorliegenden Edukte setzen sich beim Batch-Verfahren nicht nur zum gewünschten Produkt um, sondern treten bereits in früher Reaktionsphase bevorzugt auch in Folgereaktionen ein, die verstärkt zu Harz- und Nebenprodukten führen.

Es wurde nun überraschend gefunden, daß Alkaliphenolate und Naphtholate und/oder Alkalicarbonate mit inerten organischen Flüssigkeiten bei Raumtemperatur stabile fließ- und pumpfähige Dispersionen bilden. Es zeigte sich, daß durch Umsetzung der festen Edukte in Form solcher Dispersionen, die Selektivität der Reaktion deutlich erhöht werden kann, insbesondere wenn die Zugabe zur Reaktionsmischung über den gesamten Reaktionsverlauf ausgedehnt wird.

Gegenstand der Erfindung ist daher ein Verfahren zur Herstellung von aromatischen Hydroxycarbonsäuren bzw. deren Disalzen durch Umsetzung von Alkaliphenolaten oder Naphtholaten mit Kohlendioxid, gegebenenfalls in Gegenwart eines weiteren Alkalimetallsalzes, dadurch gekennzeichnet, daß die festen Phenolat- oder Naphtholat-Edukte sowie gegebenenfalls das Alkalimetallsalz getrennt oder gemeinsam in Form einer Dispersion in einer inerten organischen Flüssigkeit in die Reaktionsmischung absatzweise oder kontinuierlich eingebracht werden.

Kontinuierlich bedeutet im Sinne der Erfindung über den gesamten Reaktionsverlauf, wobei die Zugabe stetig oder getaktet, beispielsweise über eine Schleuse, erfolgen kann.

Das erfindungsgemäße Verfahren ermöglicht die Herstellung aromatischer Hydroxycarbonsäuren in guten Ausbeuten und hoher chemischer Selektivität. Auf zusätzliche Maßnahmen zur Steigerung der Selektivität, wie sie für die Kolbe-Schmitt-Reaktion beispielsweise in EP-A 0 053 824, EP-A 0 081 753 und EP-A 0 254 596 beschrieben sind, kann verzichtet werden.

Wärmelabile und oxidationsempfindliche Stoffe, wie die Phenolate und Naphtholate, können bei Raumtemperatur absatzweise oder kontinuierlich zudosiert werden, und müssen nicht wie bisher bei hohen Temperaturen unter Inkaufnahme von Zersetzungsreaktionen als Schmelze zudosiert werden.

Als aromatische Phenolate oder Naphtholate werden bevorzugt Verbindungen der Formel (I) eingesetzt,

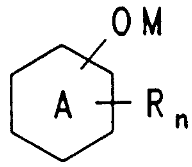

(I)

worin die Symbole und Indizes folgende Bedeutungen haben:

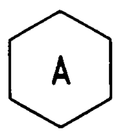 : 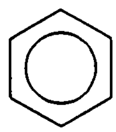 , 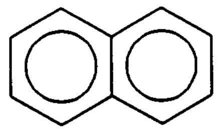 ;

M : Li, Na, K;
R : unabhängig voneinander, OM, COOM, F, Cl, Br, I, $NH_2$, $CF_3$, eine Alkylgruppe mit 1 bis 6 C-Atomen, eine Alkoxygruppe mit 1 bis 6 C-Atomen;
n : 0, 1, 2, 3, 4.

Vorzugsweise sind:

M : Na, K;
R : -OM, -COOM, eine Alkylgruppe mit 1 bis 6 C-Atomen;
n : 0, 1, 2.

Besonders bevorzugt sind:

M : K;
R : COOM, $-CH_3$;
n : 0,1.

Insbesondere bevorzugt als Verbindung der Formel (I) sind Natrium-β-naphtholat und Kalium-β-naphtholat.

Die erfindungsgemäß eingesetzten Alkaliphenolate und Naphtholate können durch Umsetzung der entsprechenden Phenole oder Naphthole mit basischen Alkaliverbindungen, wie Natriumhydroxid und Kaliumhydroxid, hergestellt werden.

Besonders vorteilhaft ist es, wenn in dem erfindungsgemäßen Verfahren nicht nur die entsprechenden Alkalimetallphenolate oder Naphtholate, sondern auch basische Salze, wie Carbonate, Hydrogencarbonate, Alkylcarboxylate, Alkoholate und Alkylate, als Dispersion in einer inerten organischen Flüssigkeit kontinuierlich zugesetzt werden.

Als Alkalisalze werden in dem erfindungsgemäßen Verfahren vorzugsweise Alkalicarbonate und Hydrogencarbonate, d. h. $Li_2CO_3$, $LiHCO_3$, $Na_2CO_3$, $NaHCO_3$, $K_2CO_3$, $KHCO_3$, $Rb_2CO_3$, $RbHCO_3$, $Cs_2CO_3$, $CsHCO_3$, eingesetzt. Besonders bevorzugt ist der Einsatz von $Na_2CO_3$ und $K_2CO_3$, insbesondere von $K_2CO_3$.

Die erfindungsgemäß verwendeten Alkalisalze sollten vorzugsweise wenig Feuchtigkeit enthalten, insbesondere weniger als 0,5 Gew.-% Wasser. Eine geringe Menge von Feuchtigkeit ist aber nicht kritisch.

Das stöchiometrische Verhältnis von Phenolat oder Naphtholat zu Alkalisalz beträgt im allgemeinen 1:0,1 bis 1:4, bevorzugt 1:0,5 bis 1:2, besonders bevorzugt 1:1.

Die Korngröße der eingesetzten festen Edukte (Phenolat oder Naphtholat und Alkalisalz) liegt vorzugsweise unter 50 μm, besonders bevorzugt unter 10 μm, insbesondere zwischen 0,1 und 10 μm.

Als Mahlaggregate für die festen Edukte eignen sich alle Zerkleinerungsmaschinen, z. B. Mühlen, die spröde Feststoffe zu derartigen Korngrößen zerkleinern können. Besonders gute Ergebnisse werden erzielt, wenn die Feststoffe direkt mit dem Dispergiermittel naß, z. B. in Kugelmühlen, vermahlen werden.

Als Dispergiermittel zur Herstellung der erfindungsgemäßen Dispersion werden unter den Reaktionsbedingungen inerte, temperaturstabile, flüssige Stoffe oder Stoffgemische eingesetzt, wie aliphatische, alicyclische oder aromatische Kohlenwasserstoffe aus der Erdöldestillation. Insbesondere geeignet sind Leichtöl, Schweröl, vorzugsweise Kerosin, Aromaten und deren Alkylderivate, wie Toluol, Xylol, Isopropyl- oder Diisopropylnaphthalin, Diphenyl, Alkyldiphenyle,

Triphenyl und Alkyltriphenyle, sowie aliphatische und aromatische Etherverbindungen und deren Alkylderivate, wie Diphenylether, Anisol, Dicyclohexylether, und Mischungen aus denselben. Die Dispergiermittel stören die Reaktion nicht und können aus dem Reaktor abdestilliert werden oder im Anschluß an die Reaktion durch Dekantieren oder Destillieren von der Reaktionsmasse abgetrennt werden. Sie haben den Vorteil, daß sie nach Entfernung von möglicherweise gelösten oder mitgeschleppten Bestandteilen, wie β-Naphthol oder Wasser, wieder zur Erzeugung frischer Dispersionen eingesetzt werden können.

Die Eindosierung der Phenolate bzw. Naphtholate sowie des Alkalisalzes kann in einer stofflich gemeinsamen Dispersionen oder in stofflichen Einzeldispersionen erfolgen, wobei letztere Möglichkeit eine größere Variationsbreite für die Reaktion eröffnet. Zum Einbringen in die Reaktionsmischung sind alle Dosiereinrichtungen geeignet, die Dispersionen oder Pasten gegen den Druck im Reaktionsgefäß fördern können, wie Kolbenpumpen, Membranpumpen, Extruder und Exzenterschneckenpumpen.

Die Zusammensetzung der Dispersion kann gewichtsmäßig in weiten Grenzen liegen und richtet sich danach, ob sie als leichtfluides Medium oder in pastöser Form gefördert werden soll. In Bezug auf Alkaliphenolat oder Naphtholat eignet sich ein Gewichtsanteil von 5 bis 70 Gew.-%, vorzugsweise 25 bis 60 Gew.-%, insbesondere auch zwischen 40 und 55 Gew.-% in Bezug auf das Gesamtgewicht der Dispersion. Das Alkalisalz kann in den Grenzen von 5 bis 65 Gew.-%, vorzugsweise 20 bis 55 Gew.-%, insbesondere im Bereich von 25 bis 40 Gew.-%, bezogen auf das Gesamtgewicht der Dispersion, eingesetzt werden. Bei Feststoffgehalten unter 25 Gew.-% ist unter Umständen ein Absetzverhalten zu beobachten, das bereits durch moderates Rühren oder Pumpen verhindert werden kann.

Die als Dispergiermittel beschriebenen organischen Flüssigkeiten können der Reaktion auch als zusätzliche Löse- und Verdünnungsmittel zugesetzt werden, vorzugsweise in Mengen von 10 bis 300 Gew.-%, besonders bevorzugt 50 bis 150 Gew.-%, bezogen auf die festen Edukte.

Analog dem in der EP-A 0 081 753 beschriebenen Verfahren zur Abtrennung von Nebenprbdukten bei der Synthese von 2-Hydroxy-naphthalin-6-carbonsäure nach Kolbe-Schmitt kann das Dispergiermittel und/oder ein zusätzliches Verdünnungsmittel auch zur Abtrennung von Nebenprodukten verwendet werden.

Das erfindungsgemäße Verfahren wird in Gegenwart von $CO_2$ durchgeführt. Das $CO_2$ kann als Gasatmosphäre über der Reaktionsmischung vorliegen oder auf, bzw. auch direkt in die Mischung gedrückt werden. Um die Reaktion erfolgreich ablaufen zu lassen, werden mindestens stöchiometrische Mengen, bezogen auf das Phenolat bzw. Naphtholat, an $CO_2$ benötigt.

Das erfindungsgemäße Verfahren wird bei einem Druck von 1 bis 50 bar, vorzugsweise 5 bis 20 bar und besonders bevorzugt von 8 bis 15 bar betrieben, wobei der Druck bei der Reaktionstemperatur gemeint ist.

Es kann technisches Kohlendioxid verwendet werden, d. h., geringe Mengen an Fremdgasen, wie $N_2$, $CH_4$, CO, $H_2$, sind unkritisch.

Die Reaktionstemperatur kann je nach Eigenschaft der Edukte, Produkte und Löse- bder Dispergiermittel in weiten Grenzen variiert werden. Im allgemeinen wird bei Temperaturen von 150 bis 400°C, bevorzugt von 150 bis 350°C, ganz besonders bevorzugt von 200 bis 350°C und insbesondere bevorzugt von 250 bis 350°C gearbeitet.
Die Reaktionsdauer beträgt vorzugsweise zwischen 1 und 40 Stunden.

Zur Durchführung des erfindungsgemäßen Verfahrens können unterschiedliche apparative Ausführungsformen zum Einsatz gelangen, so kann beispielsweise als Reaktor ein Druckgefäß oder Kneter verwendet werden, der mit einem zur Durchmischung und $CO_2$-Begasung effizienten Rührorgan ausgerüstet und mit einer Dispersionseinspeisung verbunden ist. Dabei kann die Dispersion entweder getaktet zugepumpt oder zugedrückt bzw. kontinuierlich eindosiert werden.

In einer bevorzugten Variante des erfindungsgemäßen Verfahrens werden Phenolat oder Naphtholat und gegebenenfalls das Alkalisalz und Dispergiermittel vermahlen und die so erhaltene Dispersion kontinuierlich oder getaktet in einen vorgeheizten, mit $CO_2$ und gegebenenfalls mit Lösemittel und Alkalisalz beschickten Reaktor eingebracht. Nach Beendigung der Reaktion wird das entstandene Disalz der Hydroxycarbonsäure in die freie Säure überführt und gegebenenfalls nach den bekannten Verfahren gereinigt.

Das erfindungsgemäße Verfahren kann diskontinuierlich, semikontinuierlich oder kontinuierlich betrieben werden.

In Figur 1 ist eine mögliche Variante zur apparativen Durchführung des erfindungsgemäßen Verfahrens dargestellt.

Apparative Durchführung am Beispiel der 2-Hydroxy-naphthalin-6-carbonsäure:

2-Naphthol wird zusammen mit Kalilauge im Eindampfer (1] entwässert und im Trockner (2) von Restfeuchte befreit. Die gebildete Kaliumnaphtholatschmelze wird anschließend unter Kühlung in der Mühle (3) mit Kerosin zu einer Dispersion vermahlen und im Tank (4) bevorratet. Im Reaktor (5) wird Kerosin aus dem Behälter (12) vorgelegt und unter $CO_2$ Druck die Suspension von Kaliumnaphtholat und Kaliumcarbonat (hergestellt durch Vermahlen mit Kerosin in der Mühle (10) und im Tank (11) bevorratet) kontinuierlich dem Reaktor zugeführt. Nach erfolgter Reaktion wird die Reaktionsschmelze im Rührbehälter (6) in Wasser aufgenommen, Kerosin als Oberphase abdekantiert und mit Schwefelsäure neutral gestellt. Das ausgefallene 2-Naphthol wird in der Zentrifuge (7) abgetrennt. Die Ansäuerung und Fällung der 2-Hydroxy-naphthalin-6-carbonsäure geschieht analog in (8) und (9).

Die nach dem erfindungsgemäßen Verfahren hergestellten aromatischen Hydroxycarbonsäuren sind wichtige Zwischenprodukte zur Herstellung von Polyestern, Azofarbstoffen und Arzneimitteln.

Insbesondere stellt 2-Hydroxy-naphthalin-6-carbonsäure nicht nur einen wertvollen Synthesebaustein für Farbstoffe, Textilhilfsmittel und Arzneimittel dar (siehe z. B. EP-A 0 292 955 A), sondern auch ein wichtiges Monomeres zur Herstellung von flüssigkristallinen Polymeren mit überragenden Eigenschaften (siehe z. B. US-PS 4 393 191).

Die nachfolgenden Beispiele dienen zur Erläuterung der vorstehend beschriebenen Erfindung. Die Teile sind Gewichtsteile, die Prozentangaben stellen Gewichtsprozente dar, sofern nicht anders vermerkt.

Beispiele

Beispiel 1

Ein Edelstahl-Druckautoklav wird mit 100 Teilen Kerosin und 38 Teilen Kaliumcarbonat befüllt und auf 280°C aufgeheizt. Danach werden 10 bar Kohlendioxid aufgedrückt. Über eine Dosierpumpe werden im Verlauf von 5 Stunden 50 Teile Kalium-β-naphtholat als Suspension in Kerosin eindosiert. Kerosin wird dabei teilweise aus dem Reaktionsgefäß bei vermindertem Druck abdestilliert, kondensiert und ausgeschleust. Nach dem Abkühlen wird die Reaktionsmischung in Wasser aufgenommen und Kerosin als Oberphase abdekantiert. Nach Ansäuerung mit Schwefelsäure auf einen pH-Wert von 7 kann unumgesetztes β-Naphthol gefällt und abgetrennt werden. Bei weiterer Ansäuerung auf pH 4 fällt zunächst die 2-Hydroxy-naphthalin-6-carbonsäure und bei pH 1 schließlich die übrigen Säuren aus. Die 2-Hydroxy-naphthalin-6-carbonsäure wird in einer Ausbeute von 67 % bezogen auf eingesetztes K-β-Naphtholat erhalten. Daneben fallen 23 % unumgesetztes β-Naphtholat und geringe Anteile der 2-Hydroxynaphthalin-3-carbonsäure und 2-Hydroxy-naphthalin-3,6-dicarbonsäure an.

Beispiel 2

Ein Edelstahl-Druckautoklav wird mit 50 Teilen Kerosin und 38 Teilen Kaliumcarbonat befüllt und auf 280°C aufgeheizt. Danach werden 10 bar Kohlendioxid aufgedrückt. Über eine Dosierpumpe wird im Verlauf von 5 Stunden eine Mischung von 50 Teilen Kalium-β-naphtholat und 38 Teilen Kaliumcarbonat als Suspension in Kerosin eindosiert. Kerosin wird dabei teilweise aus dem Reaktionsgefäß bei vermindertem Druck abdestilliert, kondensiert und ausgeschleust. Nach dem üblichen Aufarbeiten wird 2-Hydroxy-naphthalin-6-carbonsäure in einer Ausbeute von 76 % bezogen auf eingesetztes K-Naphtholat erhalten. Daneben fallen 14 % unumgesetztes β-Naphthol und geringe Anteile der 2-Hydroxy-naphthalin-3-carbonsäure sowie 2-Hydroxy-naphthalin-3,6-dicarbonsäure an.

Beispiel 3

Ein Edelstahl-Druckautoklav wird mit 150 Teilen Kerosin und 32 Teilen Natriumcarbonat befüllt und auf 240°C aufgeheizt. Danach werden 10 bar Kohlendioxid aufgedrückt. Über eine Dosierpumpe wird im Verlauf von 6 Stunden 50 Teile Natrium-β-naphtholat als Suspension in Kerosin eindosiert. Kerosin wird dabei teilweise aus dem Reaktionsgefäß bei vermindertem Druck abdestilliert, kondensiert und ausgeschleust. Nach dem üblichen Aufarbeiten wird 2-Hydroxy-naphthalin-3-carbonsäure in einer Ausbeute von 65 % bezogen auf eingesetztes Na-β-Naphtholat erhalten. Daneben fallen 23 % unumgesetztes β-Naphthol und geringe Anteile der 2-Hydroxy-naphthalin-6-carbonsäure und 2-Hydroxy-naphthalin-3,6-dicarbonsäure an.

Beispiel 4

Ein Edelstahl-Druckautoklav wird mit 50 Teilen Kerosin befüllt und auf 240°C aufgeheizt. Danach werden 10 bar Kohlendioxid aufgedrückt. Über eine Dosierpumpe wird im Verlauf von 8 Stunden eine Mischung bestehend aus 50 Teilen Natrium-β-naphtholat und 32 Teilen Natriumcarbonat als Suspension in Kerosin eindosiert. Kerosin wird dabei teilweise aus dem Reaktionsgefäß bei vermindertem Druck abdestilliert, kondensiert und ausgeschleust. Nach dem üblichen Aufarbeiten wird 2-Hydroxy-naphthalin-3-carbonsäure in einer Ausbeute von 69 % bezogen auf eingesetztes Na-β-Naphtholat erhalten. Daneben fallen 20 % unumgesetztes β-Naphthol und geringe Anteile der 2-Hydroxy-naphthalin-6-carbonsäure und 2-Hydroxy-naphthalin-3,6-dicarbonsäure an.

**Patentansprüche**

1. Verfahren zur Herstellung von aromatischen Hydroxycarbonsäuren bzw. deren Disalzen durch Umsetzung von Alkaliphenolaten oder -naphtholaten mit Kohlendioxid, gegebenenfalls in Gegenwart eines weiteren Alkalimetallsalzes, dadurch gekennzeichnet, daß die festen Phenolat- oder Naphtholat-Edukte sowie gegebenenfalls das Alkalimetallsalz getrennt oder gemeinsam in Form einer Dispersion in einer inerten organischen Flüssigkeit in die

Reaktionsmischung absatzweise oder kontinuierlich eingebracht werden.

2. Verfahren nach Anspruch 1, dadurch gekennzeichnet, daß die Dispersion stetig zugegeben wird.

3. Verfahren nach Anspruch 1 oder 2, dadurch gekennzeichnet, daß die Dispersion getaktet zugegeben wird.

4. Verfahren nach einem oder mehreren der vorhergehenden Ansprüche, dadurch gekennzeichnet, daß als Phenolat oder Naphtholat eine Verbindung der Formel (I) eingesetzt wird,

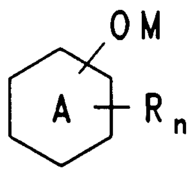

(I)

worin die Symbole und Indizes folgende Bedeutungen haben:

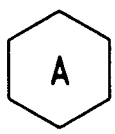 : 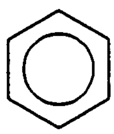 , 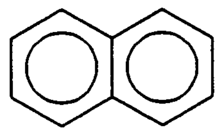 ;

M : Li, Na, K;
R : unabhängig voneinander, OM, COOM, F, Cl, Br, I, $NH_2$, $CF_3$, eine Alkylgruppe mit 1 bis 6 C-Atomen, eine Alkoxygruppe mit 1 bis 6 C-Atomen;
n : 0, 1, 2, 3, 4.

5. Verfahren nach Anspruch 4, dadurch gekennzeichnet, daß als Naphtholat Kalium-β-naphtholat eingesetzt wird.

6. Verfahren nach einem oder mehreren der vorhergehenden Ansprüche, dadurch gekennzeichnet, daß als Alkalimetallsalz Kaliumcarbonat eingesetzt wird.

7. Verfahren nach einem oder mehreren der vorhergehenden Ansprüche, dadurch gekennzeichnet, daß die inerte organische Flüssigkeit ein aliphatischer oder aromatischer Kohlenwasserstoff oder Ether bzw. eine Mischung aus mindestens zwei solcher Verbindungen ist.

8. Verfahren nach einem oder mehreren der vorhergehenden Ansprüche, dadurch gekennzeichnet, daß die Dispersion 5 bis 50 Gew.-% ,bezogen auf die gesamte Dispersion, an Alkalimetallsalz enthält.

9. Verfahren nach einem oder mehreren der vorhergehenden Ansprüche, dadurch gekennzeichnet, daß die Dispersion 5 bis 70 Gew.-%, bezogen auf die gesamte Dispersion, an Phenolat oder Naphtholat enthält.

10. Verfahren nach einem oder mehreren der vorhergehenden Ansprüche, dadurch gekennzeichnet, daß die eingesetzten Feststoffe eine mittlere Korngröße von weniger als 50 µm haben.

11. Verfahren nach einem oder mehreren der vorhergehenden Ansprüche, dadurch gekennzeichnet, daß Alkalimetallsalz und Phenolat bzw. Naphtholat in Form einer gerneinsamen Dispersion in das Reaktionsgemisch eingebracht werden.

12. Verfahren nach einem oder mehreren der vorhergehenden Ansprüche 1 bis 12, dadurch gekennzeichnet, daß Alkalimetallsalz und Phenolat bzw. Naphtholat in Form getrennter Dispersionen in das Reaktionsgemisch eingebracht werden.

## Claims

1. A process for preparing aromatic hydroxy carboxylic acids or di-salts thereof by reaction of alkali metal phenolates or naphtholates with carbon dioxide, in the presence or absence of a further alkali metal salt, which comprises introducing the solid phenolate or naphtholate starting materials and, if desired, the alkali metal salt into the reaction mixture batchwise or continuously, separately or together, in the form of a dispersion in an inert organic liquid.

2. The process as claimed in claim 1, wherein the dispersion is added steadily.

3. The process as claimed in claim 1 or 2, wherein the dispersion is added in a pulsed manner.

4. The process as claimed in one or more of the preceding claims, wherein the phenolate or naphtholate used is a compound of the formula (I)

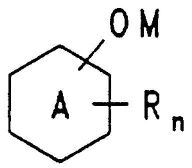

(I)

where the symbols and indices have the following meanings:

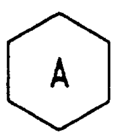 : 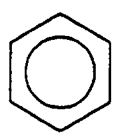 , 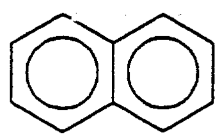 ;

M : Li, Na, K;
R : independently of one another, OM, COOM, F, Cl, Br, I, $NH_2$, $CF_3$, an alkyl group having from 1 to 6 carbon atoms, an alkoxy group having from 1 to 6 carbon atoms;
n : 0, 1, 2, 3, 4.

5. The process as claimed in claim 4, wherein the naphtholate used is potassium β-naphtholate.

6. The process as claimed in one or more of the preceding claims, wherein the alkali metal salt used is potassium carbonate.

7. The process as claimed in one or more of the preceding claims, wherein the inert organic liquid is an aliphatic or aromatic hydrocarbon or ether or a mixture of at least two such compounds.

8. The process as claimed in one or more of the preceding claims, wherein the dispersion comprises from 5 to 50% by weight, based on the total dispersion, of the alkali metal salt.

9. The process as claimed in one or more of the preceding claims, wherein the dispersion comprises from 5 to 70% by weight, based on the total dispersion, of phenolate or naphtholate.

10. The process as claimed in one or more of the preceding claims, wherein the solids used have an average particle size of less than 50 μm.

11. The process as claimed in one or more of the preceding claims, wherein the alkali metal salt and phenolate or naphtholate are introduced into the reaction mixture in the form of a common dispersion.

12. The process as claimed in one or more of the preceding claims 1 to 12, wherein the alkali metal salt and phenolate

or naphtholate are introduced into the reaction mixture in the form of separate dispersions.

## Revendications

1. Procédé pour la préparation d'acides hydroxycarboxyliques aromatiques, respectivement de leurs disels, par réaction de phénolates ou de naphtolates alcalins avec l'anhydride carbonique, éventuellement en présence d'un autre sel de métal alcalin, caractérisé en ce que l'on introduit dans le mélange réactionnel les produits de départ solides, le phénolate ou le naphtolate, ainsi qu'éventuellement le sel de métal alcalin, en continu ou en discontinu, séparément ou conjointement sous forme d'une dispersion dans un liquide organique inerte.

2. Procedé selon la revendication 1, caractérisé en ce que l'on ajoute la dispersion en continu.

3. Procédé selon la revendication 1 ou 2, caractérisé en ce que l'on ajoute la dispersion en discontinu.

4. Procédé selon une ou plusieurs des revendications précédentes, caractérisé en ce que l'on utilise comme phénolate ou naphtolate un composé de formule (I)

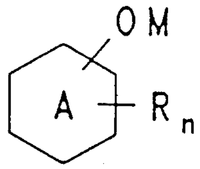

(I)

dans laquelle les symboles et les indices ont les significations suivantes :

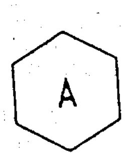 : 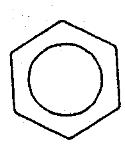 , 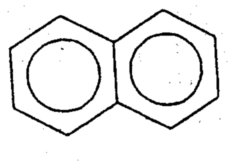 ;

M : Li, Na, K ;
R : indépendamment l'un de l'autre représente OM, COOM, F, Cl, Br, I, $NH_2$, $CF_3$, un groupe alkyle avec 1 à 6 atomes de carbone, un groupe alcoxy avec 1 à 6 atomes de carbone ;
n : vaut 0, 1, 2, 3, 4.

5. Procédé selon la revendication 4, caractérisé en ce que l'on utilise comme naphtolate le β-naphtolate de potassium.

6. Procédé selon une ou plusieurs des revendications précédentes, caractérisé en ce que l'on utilise comme sel de métal alcalin le carbonate de potassium.

7. Procédé selon une ou plusieurs des revendications précédentes, caractérisé en ce que le liquide organique inerte est un hydrocarbure aliphatique ou aromatique ou un éther, respectivement un mélange d'au moins deux de ces composés.

8. Procédé selon une ou plusieurs des revendications précédentes, caractérisé en ce que la dispersion contient de 5 à 50 % en poids de sel de métal alcalin par rapport à la totalité de la dispersion.

9. Procédé selon une ou plusieurs des revendications précédentes, caractérisé en ce que la dispersion contient de 5 à 70 % en poids de phénolate ou de naphtolate par rapport à la totalité de la dispersion.

10. Procédé selon une ou plusieurs des revendications précédentes, caractérisé en ce que les matières solides utilisées ont une granulométrie moyenne inférieure à 50 µm.

**11.** Procédé selon une ou plusieurs des revendications précédentes, caractérisé en ce que le sel de métal alcalin et le phénolate, respectivement le naphtolate sont introduits sous forme d'une dispersion commune dans la mélange réactionnel.

**12.** Procédé selon une ou plusieurs des revendications précédentes 1 à 12, caractérisé en ce que le sel de métal alcalin et le phénolate, respectivement le naphtolate, sont introduits sous forme de dispersions séparées dans le mélange réactionnel.

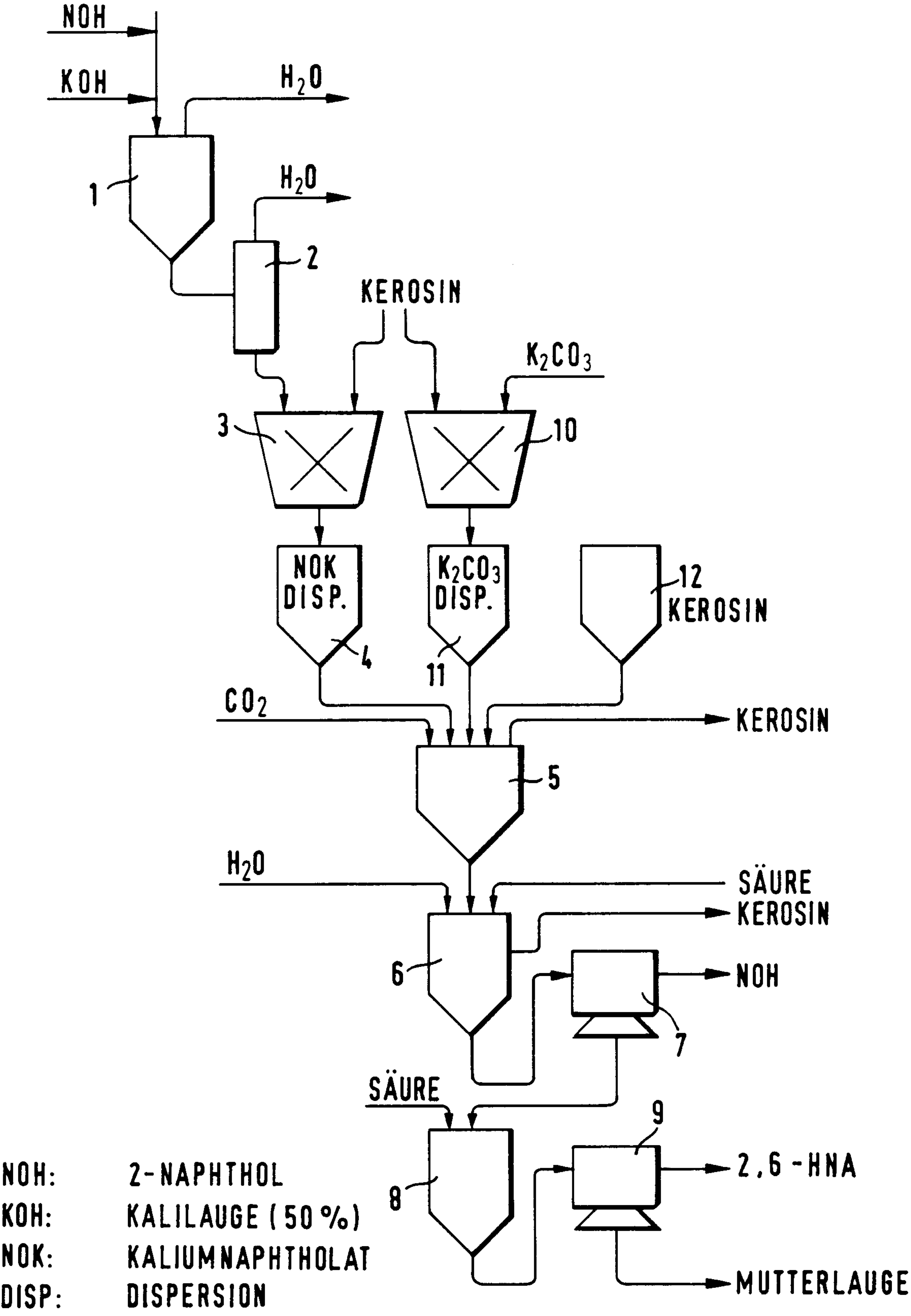


NOH: 2-NAPHTHOL
KOH: KALILAUGE (50 %)
NOK: KALIUMNAPHTHOLAT
DISP: DISPERSION
2,6-HNA: 2-HYDROXYNAPHTHALIN 6-CARBONSÄURE